# FASCICULE DE BREVET EUROPEEN

(11) **EP 3 481 364 B1**
(45) Date de publication et mention de la délivrance du brevet: **13.08.2025**
(21) Numéro de dépôt: 17745433.7
(22) Date de dépôt: 06.07.2017
(51) Int. Cl.: A61K 8/31, A61Q 9/04, A61K 8/81, A61K 8/92

(54) **COMPOSITION POUR EPILATION SANS BANDE**
ZUSAMMENSETZUNG ZUR STREIFENFREIEN HAARENTFERNUNG
NO STRIP DEPILATION COMPOSITION

(30) Priorité: 07.07.2016 FR 1656555
(43) Date de publication de la demande: 15.05.2019
(73) Titulaire: S.A. Thalgo TCH, 83520 ROQUEBRUNE-SUR-ARGENS (FR)
(72) Inventeur: LIARD, Alexis, 92200 NEUILLY SUR SEINE (FR)
(74) Mandataire: Casalonga
(86) Numéro de dépôt international: PCT/FR2017/051844
(87) Numéro de publication internationale: WO 2018/007760

(56) Documents cités:
- WO-A1-98/28015
- FR-A1- 2 751 872
- FR-A1- 2 751 873
- FR-A1- 2 940 070
- US-A1- 2010 256 274
- US-A1- 2013 150 867
- US-A1- 2016 046 736
- DATABASE GNPD [online] MINTEL; July 2009 (2009-07-01), "No strip wax", XP002764538, Database accession no. 1104693

## Description

### Contexte technique

L'invention s'inscrit dans le domaine des compositions épilatoires par arrachage des poils notamment à usage humain et en particulier, mais sans que cela soit limitatif, à usage professionnel.

On connait des compositions à base essentiellement de colophane, qui est un résidu solide de distillation de produits issus d'arbres résineux, notamment le pin. La colophane est une résine à propriété tackifiante (c'est-à-dire ayant des propriétés d'adhésion). La colophane est la plus grande fraction de ces compositions et est mélangée seulement avec de petites fractions de produits gras tels que des huiles ou de la lanoline et des cires. Ces compositions nécessitent, avant leur application, d'être longuement chauffées avant d'être suffisamment molles pour être appliquées sur la peau. Dans certaines situations, la température obtenue est trop élevée, ce qui est dangereux ou désagréable pour la personne à épiler. Par ailleurs, l'épaisseur de composition à appliquer est nécessairement importante (5 mm par exemple), du fait de la faible tenue mécanique de la composition une fois étalée. Ces compositions sont par contre réutilisables après filtration.

On connait aussi des compositions, contenant toujours de la colophane, mais comprenant aussi des huiles dans des proportions plus élevées que dans l'exemple précédent, et comprenant éventuellement des sucres ou des cires, de point de ramollissement adapté (ramollissement à température plus basse que les compositions précédentes), et qui nécessitent un chauffage moins important. Ces compositions imposent par contre l'utilisation d'un film support (bande de papier ou de coton / bande cellulosique ou de plastique, tel que du film cellophane) pour les retirer une fois qu'elles sont appliquées sur la peau, là encore du fait d'une faible tenue mécanique de la composition. Ces compositions ne sont pas réutilisables, et sont jetées après utilisation. On connait des compositions de ce type par exemple à travers le document FR2577134. D'autres compositions de ce type utilisant à la place de la colophane une résine synthétique sont également connues du document WO2008/103817. Les résines synthétiques sont décrites plus loin dans le présent texte.

Dans ce contexte l'invention porte plus précisément sur une composition pour épilation par arrachage des poils ne nécessitant pas de bande de papier ou de plastique à appliquer. La composition a des propriétés mécaniques qui sont telles que l'utilisation d'une telle bande est en effet inutile. Néanmoins, elle peut être déposée en couche mince.

On connait des compositions de ce type par exemple à travers les documents FR2617708, FR2637498, FR2751872 et FR2751873.

De manière générale, de telles compositions contiennent en premier lieu une résine à propriété tackifiante et un composé macromoléculaire apportant des propriétés mécaniques. D'autres constituants sont présents dans la composition, comme notamment des adoucissants ou assouplissants, tels des cires ou des huiles.

La résine à propriété tackifiante est souvent une colophane, c'est-à-dire, comme évoqué plus haut, une résine d'origine naturelle. Les molécules de la colophane comportent des atomes d'oxygène et ont une certaine polarité. Il peut s'agir d'une colophane non modifiée (acide) ou modifiée, par hydrogénation, estérification, ou dimérisation. Alternativement, une résine synthétique, issue de coupes de produits pétroliers, peut être utilisée. Il s'agit là d'un ensemble de molécules carbonées généralement moins polaires que la colophane. On connait des résines synthétiques appelées C5 (copolymères à base de monomères diéniques - aliphatiques - comme le pentadiène, le methylbutène, le dicyclopentadiène, le cyclopentadiène et le cyclopentène), la plupart du temps du cyclopentadiène, et des résines synthétiques appelées C9 (copolymères à base de monomères aromatiques comme le vinyltoluène, le dicyclopentadiène, l'indène, le methylstyrène, le styrène et le methylindène), la plupart du temps un copolymère styrène/méthylstyrène/indène. Ces résines existent sous différents grades et peuvent aussi être modifiées notamment par hydrogénation.

La résine, naturelle ou synthétique, est en général le composé présent dans les plus grandes proportions, en poids, dans la composition.

Le composant macromoléculaire apportant des propriétés mécaniques peut être une cire, c'est-à-dire un ester d'acide gras. Il est en général présent dans des proportions, en poids, qui sont plus faibles que celles de la résine. Il existe des cires animales, végétales ou minérales qui peuvent être utilisées, un exemple étant la cire d'abeille. Elles ont notamment un certain comportement plastique. Le composant macromoléculaire peut aussi être un élastomère, souvent meilleur marché, et qui peut avoir de meilleures propriétés mécaniques, notamment en termes de résistance à la traction et de flexibilité ou souplesse, par l'absence de comportement vitreux dans la gamme de température utilisée. L'introduction de ces élastomères par mélange, dans la résine qui a un comportement initialement vitreux, permet d'apporter une tendance amorphe. Il peut notamment s'agir d'un caoutchouc naturel ou d'un polymère ou copolymère synthétique comme un copolymère éthylène/vinyle acétate (EVA).

Les compositions de cette catégorie comprennent aussi des adoucissants et des assouplissants, tels que des graisses, des huiles ou des cires, qui participent également à la régulation rhéologique et à la modulation du temps de figeage.

Ces compositions s'utilisent par chauffage conduisant à son ramollissement par exemple autour de 55°C puis par application de la composition sur les parties du corps à épiler, à une température adaptée au confort de la personne à épiler, qui ne doit évidemment pas ressentir une température trop élevée.

Les compositions évoquées ci-dessus peuvent être déposées sous forme de couches assez fines, et retirées ensuite par pelage, après un court temps de solidification et de refroidissement permettant la prise des poils.

Les compositions sont appréciées par les personnes subissant l'épilation (par exemple des clients et clientes de salons de beauté) ainsi que par les personnes pratiquant l'épilation (par exemple les esthéticiens et esthéticiennes de salons de beauté). Les critères qui font qu'une composition est appréciée sont entre autres sa capacité à enlever les poils (aussi appelée « capacité d'arrachage »), son absence de casse (aussi nommée « propriétés mécaniques ») quand elle est manipulée sous forme de couche, ou la température ressentie sur la peau. Certains utilisateurs peuvent accorder une plus grande valeur à certains critères, et il est donc utile de disposer d'une variété de compositions différentes.

Malgré la présence sur le marché de divers produits, il reste des marges de progression pour offrir aux utilisateurs et utilisatrices des solutions qui les satisfassent tous, en fonction de leurs préférences, ou qui simplement leur permettent de changer de produit tout en conservant des propriétés satisfaisantes.

De plus jusqu'ici les élastomères étaient des composés seulement utilisés pour leurs propriétés mécaniques (flexibilité et résistance à la traction) mais très peu pour leur potentiel d'adhésion. Il en résultait une possibilité de rechercher une augmentation des capacités d'adhésion par évolution des compositions.

Il a donc été décidé de lancer des nouvelles recherches dans le domaine.

### Définition de l'invention

Pour répondre à cette problématique, il est proposé une composition adhésive comprenant une fraction principale de résine tackifiante synthétique et une fraction d'au moins un composant macromoléculaire. Les compositions selon l'invention sont définies dans le jeu de revendications.

Le composant macromoléculaire est un copolymère éthylène / octène, l'octène étant une alpha-oléfine.

Selon différentes caractéristiques avantageuses mais facultatives :
- le copolymère est un copolymère produit par catalyse avec un métallocène ;
- le copolymère est un copolymère obtenu par copolymérisation coordinative ;
- le copolymère est caractérisé par un indice de fluidité à chaud de plus de 100 g/10 min (2.16 kg@190°C), ou plus de 250 g/10 min (2.16 kg@190°C);
- le copolymère est caractérisé par un indice de fluidité à chaud de moins de 800 g/10 min (2.16 kg@190°C), ou moins de 580 g/10 min (2.16 kg@190°C);
- le copolymère est caractérisé par une densité comprise entre 0,86 et 0,91 g/cm3, ou entre 0.868 et 0.897 g/cm3 ou entre 0.870 et 0.880 g/cm3 ;
- la composition ne contient pas de copolymère éthylène / acétate de vinyle, le copolymère éthylène / octène étant présent dans la composition à hauteur d'une fraction en poids comprise entre 5 et 15 % ;
- ou au contraire la composition comprend en plus des copolymères ethylène / octène une fraction d'un copolymère éthylène / acétate de vinyle, le copolymère éthylène / octène et le copolymère éthylène acétate de vinyle étant présents ensemble dans la composition à hauteur d'une fraction en poids comprise entre 5 et 15 % ;
- la résine tackifiante synthétique est par exemple du type cyclopentadiène (C5), par exemple hydrogénée ;
- la résine tackifiante synthétique est présente à hauteur d'au moins 50 % en poids, voire plus de 60 % en poids, ou plus de 70% en poids.

L'invention vise aussi un procédé d'épilation comprenant l'application d'une couche d'une composition adhésive selon les revendications comprenant une fraction principale de résine tackifiante synthétique et une fraction d'au moins un composant macromoléculaire, l'application se faisant alors que la composition a été chauffée, puis le pelage de la couche sans l'utilisation d'une bande, une fois que la composition a refroidi.

### Description détaillée

Une formule de référence 1 est comparée à une formule 2 dans laquelle les composants élastomères (copolymères EVA à 28% d'acétate de vinyle) présents dans la formule 1 sont remplacés par un copolymère d'éthylène octène, l'octène étant une alpha-oléfine. Une résine tackifiante synthétique polycyclopentadiène hydrogénée est utilisée.

La formule 1 est indiquée dans le tableau ci-dessous :

| Résine C5 hydrogénée | Composant majoritaire (>50%) |
|---|---|
| Cires synthétiques | De l'ordre de 10 à 20 % |
| EVA (28% de VA) (deux composés différents) | Total des EVA : de l'ordre de 10 %, entre 5 et 15 % |
| total | 100% |

Les fractions sont indiquées en poids des composants rapportés au poids de l'ensemble de la composition. C'est aussi le cas dans les autres formules du présent document.

La formule 2 est indiquée dans le tableau ci-dessous :

| | |
|---|---|
| Résine C5 hydrogénée | Comme en formule 1 |
| Cires synthétiques | Comme en formule 1 |
| Copolymère ethylène / octène (Affinity GA 1950) | Autant que EVA dans la formule 1 |
| | De l'ordre de 10 %, entre 5 et 15% |
| total | 100% |

Le composant copolymère ethylène/octène Affinity GA 1950 est disponible chez Dow Chemical. Il est caractérisé par un indice de fluidité à chaud de 500 g/10 min (2.16 kg@190°C) et une densité de 0.874. Il a une résistance à la traction de 1.76 Mpa.

On peut utiliser d'autres grades de copolymères éthylène/octène, avec par exemple des indices de fluidité à chaud de plus de 100 g/10 min (2.16 kg@190°C), ou plus de 250 g/10 min (2.16 kg@190°C).

Le copolymère peut aussi avoir une densité comprise entre 0,86 et 0,91 g/cm3, 0.868 et 0.897 g/cm3, ou entre 0.870 et 0.880 g/cm3.

Les deux cires utilisées présentent à titre illustratif les caractéristiques suivantes : point de figeage 58 à 80°C.

La résine est une résine C5 hydrogénée de point de ramollissement entre 85 et 100°C et de masse moléculaire de 450 à 590 g/mol.

Les EVA utilisés à titre de référence ont des indices de fluidité à chaud respectivement de 100 et 500 g/10 min (2.16 kg@190°C) et des densités toutes deux de 950 kg/m3. Il s'agit, comme déjà mentionné, d'EVA à 28% d'acétate de vinyle.

Ces compositions sont appliquées par des spécialistes de l'épilation sur des personnes à épiler, dans le cadre de tests en aveugle. La composition, initialement en granulés, est chauffée dans un chauffe-cire jusqu'à former une pate tiède. Le chauffage se fait entre 50°C et 75°C, et préférablement 58°C à 68°C. Puis la pâte tiède est appliquée par exemple avec une spatule en bois pour former une fine couche allongée de composition qui refroidit, emprisonne les poils ou les colle et durcit.

Cette couche, une fois rigidifiée par son refroidissement spontané est saisie à l'une de ses extrémités et retirée avec rapidité dans le sens contraire de la pousse des poils, par une opération qualifiée de pelage. Le pelage est ainsi fait entre 20°C et 38°C, souvent entre 25°C et 35°C.

La formule 2 a des propriétés d'arrachage et des propriétés mécaniques (absence de casse), ainsi que des propriétés olfactives du produit qui sont améliorées par rapport à la formule 1.

L'alpha-oléfine utilisée ci-dessus est une apha-oléfine qui n'est pas un propylène.

Il s'agit d'un octène.

On s'intéresse en particulier, sans néanmoins que cela constitue une limitation de l'invention, à des copolymères de type plastomère, ayant un comportement plus dissipatif que les EVA utilisés jusqu'ici.

Les mêmes essais ont été réalisés avec des compositions à base de résine C9 (résines tackifiantes aromatiques) hydrogénées.

La résine utilisée est une résine C9 hydrogénée, de point de ramollissement compris entre 85 et 100°C et masse moléculaire de 600 à 800 g/mol.

La formule 3 est indiquée dans le tableau ci-dessous. Il s'agit d'une formule avec un copolymère éthylène acétate de vinyle (EVA).

| Résine C9 hydrogénée | Composant majoritaire (>50%) |
|---|---|
| Cires synthétiques | De l'ordre de 10 à 20% |
| Cire d'abeille | Entre 5 et 10 % |
| Huile de paraffine | Entre 1 et 3 % |
| EVA (28% VA) (deux composés différents) | De l'ordre de 10 %, entre 5 et 15 % |
| Parfum | Faible proportion (moins de 2%) |
| total | 100% |

La formule 4 est indiquée dans le tableau ci-dessous. Il s'agit d'une formule avec copolymères ethylène / octène

| | |
|---|---|
| Résine C9 hydrogénée | Comme en formule 3 |
| Cires synthétiques | Comme en formule 3 |
| Cire d'abeille | Comme en formule 3 |
| Huile de paraffine | Comme en formule 3 |
| Copolymère éthylène octène (Affinity GA 1950) | Comme l'EVA en formule 3 |
| Parfum | Comme en formule 3 |
| total | 100% |

Les copolymères d'éthylène octène mènent à une composition qui a des propriétés au moins aussi bonnes que celles de la composition comprenant des EVA. Il est vraisemblable qu'une amélioration est possible si les formules sont modifiées tout en restant dans le cadre de l'invention pour obtenir un résultat optimal en utilisant des copolymères éthylène octène.

Des expériences ont de plus été conduites avec les copolymères ethylène/octène Affinity GA 1950 mélangés dans une même composition avec les EVA selon les formules suivantes :
Formule 5 :

| Résine C9 hydrogénée | Composant majoritaire (>50%) |
|---|---|
| Cires synthétiques | De l'ordre de 10 à 20% |
| Cire d'abeille | Entre 5 et 10 % |
| Huile de paraffine | Entre 1 et 3 % |
| Copolymère éthylène / octène (Affinity GA 1950) | De l'ordre de 6 %, entre 2 et 12 % |
| EVA (28% VA) | De l'ordre de 4%, entre 2 et 10 % |
| Pigments | Faible proportion (moins de 2%) |
| Total | 100% |

Formule 6 :

| Résine C5 hydrogénée | Composant majoritaire (>50%) |
|---|---|
| Cires synthétiques | De l'ordre de 10 à 20 % |
| Copolymère éthylène / octène (Affinity GA 1950) | De l'ordre de 5%, entre 2 et et 12% |
| EVA (28% de VA) | De l'ordre de 5 %, entre 2 et 12 % |
| total | 100% |

Les résultats ont montré une amélioration des propriétés d'arrachage et des propriétés mécaniques (absence de casse), ainsi qu'une diminution des résidus et de l'odeur, par rapport à la composition similaire utilisant uniquement des EVA.

**En conclusion,** les copolymères d'éthylène / octène améliorent les performances des compositions, notamment à base de résines synthétiques, notamment hydrogénées, et offrent une nouvelle gamme de produits aux propriétés modifiées. Ces résultats intéressants ne sont pas expliqués au vu de l'art antérieur et étaient inattendus.

Les EVA (à 28% de VA) et les copolymères d'éthylène octène peuvent également agir en synergie et apporter au produit des propriétés d'arrachage et des propriétés mécaniques intéressantes par rapport aux produits de l'état de la technique.

Les copolymères d'éthylène octène utilisés ci-dessus constituent un cas particulier de composants macromoléculaires de la classe des copolymères de deux monomères ou plus pris dans le groupe comprenant l'éthylène et les alpha oléfines.

On privilégie pour l'application décrite des composants à faible cristallinité, voire amorphes. Ils sont avantageusement obtenus par polymérisation coordinative avec un catalyseur de Zigler Natta, par exemple un catalyseur métallocène.

## Revendications

1. Composition adhésive pour l'épilation sans bande comprenant une fraction principale de résine tackifiante synthétique et une fraction d'au moins un composant macromoléculaire, **caractérisée en ce que** le au moins un composant macromoléculaire comprend un copolymère d'éthylène et d'octène, l'octène étant une alpha-oléfine, la composition étant sans copolymère éthylène acétate de vinyle, le copolymère éthylène / octène étant présent dans la composition à hauteur d'une fraction en poids comprise entre 5 et 15%, et la résine tackifiante synthétique étant présente à hauteur d'au moins 50 % en poids.

2. Composition adhésive pour l'épilation sans bande comprenant une fraction principale de résine tackifiante synthétique et une fraction d'au moins un composant macromoléculaire, **caractérisée en ce que** le au moins un composant macromoléculaire comprend un copolymère éthylène / octène, l'octène étant une alpha-oléfine, la composition comprenant de plus une fraction d'un copolymère éthylène acétate de vinyle, le copolymère éthylène / octène et le copolymère éthylène acétate de vinyle étant présents ensemble dans la composition à hauteur d'une fraction en poids comprise entre 5 et 15%, et la résine tackifiante synthétique étant présente à hauteur d'au moins 50 % en poids.

3. Composition adhésive selon la revendication 1 ou la revendication 2, dans laquelle la résine tackifiante synthétique est présente à hauteur d'au moins 60 % en poids.

4. Composition adhésive selon l'une des revendications 1 à 3, dans lequel le copolymère est un copolymère obtenu par polymérisation coordinative.

5. Composition adhésive selon l'une des revendications 1 à 4, dans lequel la polymérisation est catalysée par un métallocène.

6. Composition adhésive selon l'une des revendications 1 à 12, dans laquelle la résine tackifiante synthétique est une résine du type cyclopentadiène.

7. Procédé d'épilation comprenant l'application d'une couche d'une composition adhésive selon l'une des revendications 1 à 7, l'application se faisant alors que la composition a été chauffée, puis le pelage de la couche sans l'utilisation d'une bande, une fois que la composition a refroidi.

8. Procédé d'épilation selon la revendication 7 dans lequel la résine tackifiante synthétique est présente à hauteur d'au moins 60 % en poids.

## Patentansprüche

1. Haftzusammensetzung für die streifenfreie Haarentfernung, umfassend einen Hauptanteil eines synthetischen klebrigmachenden Harzes und einen Anteil von mindestens einer makromolekularen Komponente, **dadurch gekennzeichnet, dass** die mindestens eine makromolekulare Komponente ein Copolymer von Ethylen und Octen umfasst, wobei Octen ein alpha-Olefin ist, wobei die Zusammensetzung
frei von Ethylen-Vinylacetat-Copolymer ist, das Ethylen/Octen-Copolymer in der Zusammensetzung in einem Gewichtsanteil zwischen 5 und 15 % vorhanden ist und das synthetische klebrigmachende Harz in einem Anteil von mindestens 50 Gew.-% vorhanden ist.

2. Haftzusammensetzung für die streifenfreie Haarentfernung, umfassend einen Hauptanteil eines synthetischen klebrigmachenden Harzes und einen Anteil von mindestens einer makromolekularen Komponente, **dadurch gekennzeichnet, dass** die mindestens eine makromolekulare Komponente ein Ethylen/Octen-Copolymer umfasst, wobei das Octen ein alpha-Olefin ist, wobei die Zusammensetzung
ferner einen Anteil eines Ethylen-Vinylacetat-Copolymers umfasst, wobei das Ethylen/Octen-Copolymer und das Ethylen-Vinylacetat-Copolymer zusammen in der Zusammensetzung in einem Gewichtsanteil zwischen 5 und 15 % vorhanden sind und das synthetische klebrigmachende Harz in einem Anteil von mindestens 50 Gew.-% vorhanden ist.

3. Haftzusammensetzung nach Anspruch 1 oder Anspruch 2, wobei das synthetische klebrigmachende Harz in Höhe von mindestens 60 Gew.-% vorhanden ist.

4. Haftzusammensetzung nach einem der Ansprüche 1 bis 3, wobei das Copolymer ein durch koordinative Polymerisation erhaltenes Copolymer ist.

5. Haftzusammensetzung nach einem der Ansprüche 1 bis 4, wobei die Polymerisation durch ein Metallocen katalysiert wird.

6. Haftzusammensetzung nach einem der Ansprüche 1 bis 12, wobei das synthetische klebrigmachende Harz ein Harz des Typs Cyclopentadien ist.

7. Haarentfernungsverfahren, umfassend das Auftragen einer Schicht einer Haftzusammensetzung nach einem der Ansprüche 1 bis 7, wobei das Auftragen erfolgt, während die Zusammensetzung erwärmt wurde, und anschließend das Abziehen der Schicht ohne Verwendung eines Streifens, nachdem die Zusammensetzung abgekühlt ist.

8. Haarentfernungsverfahren nach Anspruch 7, wobei das synthetische klebrigmachende Harz zu mindestens 60 Gew.-% vorhanden ist.

## Claims

1. Adhesive composition for strip-free hair removal comprising a main fraction of synthetic tackifying resin and a fraction of at least one macromolecular component, **characterised in that** the at least one macromolecular component comprises an ethylene and octene copolymer, the octene being an alpha-olefin, the composition
not including ethylene-vinyl acetate copolymer, the ethylene/octene copolymer being present in the composition in a fraction of between 5 and 15% by weight, and the synthetic tackifying resin being present in an amount of at least 50% by weight.

2. Adhesive composition for strip-free hair removal comprising a main fraction of synthetic tackifying resin and a fraction of at least one macromolecular component, **characterised in that** the at least one macromolecular component comprises an ethylene/octene copolymer, the octene being an alpha-olefin, the composition
further comprising a fraction of an ethylene-vinyl acetate copolymer, the ethylene/octene copolymer and the ethylene-vinyl acetate copolymer being present together in the composition in a fraction of between 5 and 15% by weight, and the synthetic tackifying resin being present in an amount of at least 50% by weight.

3. Adhesive composition according to claim 1 or claim 2, wherein the synthetic tackifying resin is present in an amount of at least 60% by weight.

4. Adhesive composition according to any one of claims 1 to 3, wherein the copolymer is a copolymer obtained by coordination polymerisation.

5. Adhesive composition according to any one of claims 1 to 4, wherein the polymerisation is catalysed by a metallocene.

6. Adhesive composition according to any one of claims 1 to 12, wherein the synthetic tackifying resin is a cyclopentadiene type resin.

7. Method for hair removal comprising applying a layer of an adhesive composition according to any one of claims 1 to 7, the application being carried out when the composition has been heated, the layer is then peeled off without the use of a strip once the composition has cooled.

8. Method for hair removal method according to claim **7,** wherein the synthetic tackifying resin is present in an amount of at least 60% by weight.
